(19) Europäisches Patentamt / European Patent Office / Office européen des brevets

(11) **EP 2 234 600 B1**

(12) **EUROPEAN PATENT SPECIFICATION**

(45) Date of publication and mention
of the grant of the patent:
**20.08.2014 Bulletin 2014/34**

(21) Application number: **08864416.6**

(22) Date of filing: **11.12.2008**

(51) Int Cl.:
*A61K 9/08* *(2006.01)*  *A61K 47/12* *(2006.01)*
*A61K 47/18* *(2006.01)*  *A61K 47/26* *(2006.01)*

(86) International application number:
**PCT/EP2008/067293**

(87) International publication number:
**WO 2009/080541 (02.07.2009 Gazette 2009/27)**

(54) **ANTIBODY FORMULATION**

ANTIKÖRPER-FORMULIERUNG

FORMULATION D'ANTICORPS

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**RS**

(30) Priority: **21.12.2007 EP 07150335**

(43) Date of publication of application:
**06.10.2010 Bulletin 2010/40**

(73) Proprietor: **F. Hoffmann-La Roche AG
4070 Basel (CH)**

(72) Inventors:
• **ADLER, Michael
79104 Freiburg (DE)**
• **MAHLER, Hanns-Christian
CH-4001 Basel (CH)**
• **WURTH, Christine
79539 Loerrach (DE)**

(74) Representative: **Klein, Thomas
F. Hoffmann-La Roche AG
Patent Department
Grenzacherstrasse 124
4070 Basel (CH)**

(56) References cited:
WO-A-98/56418        WO-A-2005/044859
WO-A-2006/044908     WO-A-2007/092772

**Description**

The present invention relates to an anti- CD20 monoclonal antibody formulation, a process for the preparation of said formulation and uses of the formulation.

Background of the Invention

The CD20 molecule (also called human B-lymphocyte-restricted differentiation antigen or Bp35) is a hydrophobic transmembrane protein with a molecular weight of approximately 35 kD located on pre-B and mature B lymphocytes (Valentine et al. (1989) J. Biol. Chem. 264(19):11282-11287; and Einfield et al. (1988) EMBO J. 7(3):711-717). CD20 is found on the surface of greater than 90% of B cells from peripheral blood or lymphoid organs and is expressed during early pre-B cell development and remains until plasma cell differentiation. CD20 is present on both normal B cells as well as malignant B cells. In particular, CD20 is expressed on greater than 90% of B cell non-Hodgkin's lymphomas (NHL) (Anderson et al. (1984) Blood 63(6): 1424-1433)) but is not found on hematopoietic stem cells, pro-B cells, normal plasma cells, or other normal tissues (Tedder et al. (1985) J, Immunol. 135(2):973- 979).

The 85 amino acid carboxyl-terminal region of the CD20 protein is located within the cytoplasm. The length of this region contrasts with that of other B cell-specific surface structures such as IgM, IgD, and IgG heavy chains or histocompatibility antigens class I1 a or ß chains, which have relatively short intracytoplasmic regions of 3, 3, 28, 15, and 16 amino acids, respectively (Komaromy et al. (1983) NAR 11:6775-6785). Of the last 61 carboxyl-terminal amino acids, 21 are acidic residues, whereas only 2 are basic, indicating that this region has a strong net negative charge. The GenBank Accession No. is NP-690605. It is thought that CD20 might be involved in regulating an early step(s) in the activation and differentiation process of B cells (Tedder et al. (1986) Eur. J. Immunol. 25 16:881-887) and could function as a calcium ion channel (Tedder et al. (1990) J. Cell. Biochem. 14D: 195).

There exist two different types of anti-CD20 antibodies differing significantly in their mode of CD20 binding and biological activities (Cragg, M.S., et al, Blood, 103 (2004) 2738-2743; and Cragg, M.S., et al, Blood, 101 (2003) 1045-1051). Type I antibodies, as Rituximab, are potent in complement mediated cytotoxicity, whereas type II antibodies, as Tositumomab (Bexxar®, B1), 11B8 and AT80, effectively initiate target cell death via caspase-independent apoptosis with concomitant phosphatidylserine exposure.

The sharing common features of type I and type II anti-CD20 antibodies are summarized in Table 1.

Table 1: Properties of type I and type II anti-CD20 antibodies

| type I anti-CD20 antibodies | type II anti-CD20 antibodies |
| --- | --- |
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity(if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Detailed Description of the Invention

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained.

"Antibody fragments" comprise a portion of a full length antibody, generally at least the antigen binding portion or the variable region thereof. Examples of antibody fragments include diabodies, single-chain antibody molecules, immunotoxins, and multispecific antibodies formed from antibody fragments. In addition, antibody fragments comprise single chain polypeptides having the characteristics of a VH chain, namely being able to assemble together with a VL chain or of a VL chain binding to the CD20 antigen, namely being able to assemble together with a VH chain to a functional antigen binding pocket.

"Antibody fragments" also comprises such fragments which per se are not able to provide effector functions (ADCC/CDC) but provide this function in a manner according to the invention after being combined with appropriate antibody constant domain(s).

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. Pharmacol. 5 (2001) 368-374). Based on such technology, human antibodies against a great variety of targets can be produced. Examples of human antibodies are for example described in Kellermann, S. A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, "specifically binding" or "binds specifically to" refers to an antibody specifically binding to the CD20 antigen. Preferably the binding affinity is of KD-value of $10^{-9}$ mol/l or lower (e.g. $10^{-10}$ mol/l), preferably with a KD-value of $10^{-10}$ mol/l or lower (e.g. $10^{-12}$ mol/l). The binding affinity is determined with a standard binding assay, such as surface plasmon resonance technique (Biacore®).

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th Ed. Public Health Service, National Institutes of Health, Bethesda, MD. (1991)) and/or those residues from a "hypervariable loop".

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms:

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al, Blood 103 (2004) 2738-2743; and Cragg, M.S., et al Blood 101 (2003) 1045-1051, see Table 2. /

Table 2: Properties of type I and type II anti-CD20 antibodies

| type I anti-CD20 antibodies | type II anti-CD20 antibodies |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity(if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

One essential property of type I and type II anti-CD20 antibody is their mode of binding. Thus type I and type II anti-CD20 antibody can be classified by the ratio of the binding capacities to CD20 on Raji cells (ATCC-No. CCL-86) of said anti-CD20 antibody compared to rituximab.

As used herein, "anti-CD20 antibody" can be either a type I or type II antibody. Preferably, it is a type II antibody.

The type I anti-CD20 antibodies have a ratio of the binding capacities to CD20 on Raji cells (ATCC-No. CCL-86) of said anti-CD20 antibody compared to rituximab of 0.8 to 1.2, preferably of 0.9 to 1.1. Examples of such type I anti-CD20 antibodies include e.g. rituximab, (WO94/11026), 1F5 IgG2a (ECACC, hybridoma; Press et al., Blood 69/2:584-591 (1987)), HI47 IgG3 (ECACC, hybridoma), 2C6 IgG1 (as disclosed in WO2005/103081), 2F2 IgG1 (as disclosed and WO 2004/035607 and WO2005/103081) and 2H7 IgG1 (as disclosed in WO 2004/056312). Preferably said type I anti-CD20 antibody is a monoclonal antibody that binds to the same epitope as rituximab. The type II anti-CD20 antibodies have a ratio of the binding capacities to CD20 on Raji cells (ATCC-No. CCL-86) of said anti-CD20 antibody compared to rituximab of 0.3 to 0.6, preferably of 0.35 to 0.55, more preferably 0.4 to 0.5. Examples of such type II anti-CD20 antibodies include e.g. tositumomab (B1 IgG2a), humanized B-Lyl antibody IgG1 (a chimeric humanized IgG1 antibody as disclosed in WO2005/044859), 11B8 IgG1 (as disclosed in WO 2004/035607), and AT80 IgG1. Preferably said type II anti-CD20 antibody is a monoclonal antibody that binds to the same epitope as humanized B-Lyl antibody (as disclosed in WO2005/044859).

The "ratio of the binding capacities to CD20 on Raji cells (ATCC-No. CCL-86) of an anti-CD20 antibodies compared to rituximab" is determined by direct immunofluorescence measurement (the mean fluorescent intensities (MFI) is measured) using said anti-CD20 antibody conjugated with Cy5 and rituximab conjugated with Cy5 in a FACSArray (Becton Dickinson) with Raji cells (ATCC-No. CCL-86), as described in Example No. 2, and calculated as follows:

$$\text{Ratio of the binding capacities to CD20 on Raji cells (ATCC-No. CCL-86)} = \frac{\text{MFI(Cy5-anti-CD20 antibody)}}{\text{MFI(Cy5-rituximab)}} \times \frac{\text{Cy5-labeling ratio(Cy5-rituximab)}}{\text{Cy5-labeling ratio(Cy5-anti-CD20 antibody)}}$$

MFI is the mean fluorescent intensity. The "Cy5-labeling ratio" as used herein means number of Cy5-label molecules per molecule antibody.

Typically said type I anti-CD20 antibody has a ratio of the binding capacities to CD20 on Raji cells (ATCC-No. CCL-86) of said first anti-CD20 antibody compared to rituximab of 0.8 to 1.2, preferably 0.9 to 1.1.

Typically said type II anti-CD20 antibody has a ratio of the binding capacities to CD20 on Raji cells (ATCC-No. CCL-86) of said second anti-CD20 antibody compared to rituximab of 0.3 to 0.6, preferably 0.35 to 0.55, more preferably 0.4 to 0.5.

In a preferred embodiment said type II anti-CD20 antibody, preferably a humanized B-Lyl antibody, has increased antibody dependent cellular cytotoxicity (ADCC).

By "antibody having increased antibody dependent cellular cytotoxicity (ADCC)" is meant an antibody, as that term is defined herein, having increased ADCC as determined by any suitable method known to those of ordinary skill in the art. One accepted in vitro ADCC assay is as follows:

1) the assay uses target cells that are known to express the target antigen recognized by the antigen-binding region of the antibody;

2) the assay uses human peripheral blood mononuclear cells (PBMCs), isolated from blood of a randomly chosen healthy donor, as effector cells;

3) the assay is carried out according to following protocol:

i) the PBMCs are isolated using standard density centrifugation procedures and are suspended at 5 X 106 cells/ml in RPMI cell culture medium;

ii) the target cells are grown by standard tissue culture methods, harvested from the exponential growth phase with a viability higher than 90%, washed in RPMI cell culture medium, labeled with 100 micro-Curies of "CI-, washed twice with cell culture medium, and resuspended in cell culture medium at a density of 1 0' cells/ml;

iii) 100 microliters of the final target cell suspension above are transferred to each well of a 96-well microtiter plate;

iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;

v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);

vi) for the spontaneous release (SR) controls, 3 additional wells in the plate containing the labeled target cells, receive 50 microliters of RPMI cell culture medium instead of the antibody solution (point iv above);

vii) the 96-well microtiter plate is then centrifuged at 50 x g for 1 minute and incubated for 1 hour at 4 C;

viii) 50 microliters of the PBMC suspension (point i above) are added to each well to yield an effector:target cell ratio of 25: 1 and the plates are placed in an incubator under 5% CO2 atmosphere at 37 C for 4 hours;

ix) the cell-free supernatant from each well is harvested and the experimentally released radioactivity (ER) is quantified using a gamma counter;

x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);

4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P. et al., Nature Biotechnol. 17:176-180 (1999) and U.S. Pat. No. 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. The assay is performed preferably with $^{51}$Cr or Eu labeled tumor cells and measurement of released $^{51}$Cr or Eu. Controls include the incubation of the tumor target cells with complement but without the antibody.

Typically type I and type II anti-CD20 antibodies of the IgG 1 isotype show characteristic CDC properties. Type I anti-CD20 antibodies have and increased CDC (if IgG1 isotype) and type II anti-CD20 antibodies have a decreased CDC (if IgG1 isotype) compared to each other. Preferably both type I and type II anti-CD20 antibodies are IgG1 isotype antibodies.

The "rituximab" antibody is a genetically engineered chimeric human gamma 1 murine constant domain containing monoclonal antibody directed against the human CD20 antigen. This chimeric antibody contains human gamma 1 constant domains and is identified by the name "C2B8" in WO94/11026 (Anderson et. al.). Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff et. al, Blood 83(2): 435-445 (1994)). Additionally, it exhibits significant activity in assays that measure antibody-dependent cellular cytotoxicity (ADCC).

The term "humanized B-Lyl antibody" refers to humanized B-Lyl antibody as disclosed in WO2005/044859, which were obtained from the murine monoclonal anti-CD20 antibody B-Lyl (variable region of the murine heavy chain (VH): SEQ ID NO: 1; variable region of the murine light chain (VL): SEQ ID NO: 2- see Poppema, S. and Visser, L., Biotest Bulletin 3: 131-139 (1987);) bychimerization with a human constant domain from IgG1 and following humanization (see WO2005/044859). These "humanized B-Lyl antibodies" are disclosed in detail in WO2005/ 044859.

. Furthermore the humanized B-Lyl antibody is preferably an IgG1 antibody. Preferably such humanized B-Lyl antibodies are glycoengineered (GE) in the Fc region according to the procedures described in WO2005/044859, WO 2004/065540, Umana, P. et al., Nature Biotechnol. 17:176-180 (1999) and WO 99/154342. Most glycoengineered humanized B-Lyl antibodies have an altered pattern of glycosylation in the Fc region, preferably having a reduced level of fucose residues. Preferably at least 40% or more (in one embodiment between 40% and 60%, in another embodiment at least 50%, and in still another embodiment at least 70% or more) of the oligosaccharides of the Fc region are non-fucosylated. Furthermore the oligosaccharides of the Fc region are preferably bisected. The "humanized B-Lyl antibody" comprises VH B-HH6 and VL B-KV1 of WO2005/044859. As used herein, said antibody is also referred to as "HuMab<CD20>". In another most preferable embodiment, said antibody has a reduced level of fucose residues as defined above and/or the oligosaccharides of the Fc region are most preferably bisected. In yet another most preferable embodiment, said antibody displays increased ADCC as defined herein.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins et al., Nature Biotechnol. 14:975-81 (1996)).

Mammalian cells are the preferred hosts for production of therapeutic glycoproteins, due to their capability to glycosylate proteins in the most compatible form for human application. (Cumming et al., Glycobiology 1:115-30 (1991); Jenkins et al., Nature Biotechnol. 14:975-81 (1996)). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serumfree media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins et al., Nature Biotechnol. 14: 975-981 (1996).

All antibodies contain carbohydrate structures at conserved positions in the heavy chain constant regions, with each isotype possessing a distinct array of N-linked carbohydrate structures, which variably affect protein assembly, secretion or functional activity. (Wright, A., and Monison, S. L., Trends Biotech. 15: 26-32 (1997)). The structure of the attached N-linked carbohydrate varies considerably, depending on the degree of processing, and can include highmannose, multiply-branched as well as biantennary complex oligosaccharides. (Wright, A., and Morrison, S. L., Trends Biotech. 15: 26-32 (1997)). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M. R. et al., Glycobiology 5(8):813-22 (1995)).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P. et al., Nature Biotechnol. 17:176-180 (1999) and U.S. Pat. No. 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M. R., et al., Glycobiology 5: 813-822 (1995); Jefferis, R., et al., Immunol. Rev. 163: 59-76 (1998); Wright, A. and Morrison, S. L., Trends Biotechnol. 15: 26-32 (1997)).

It was previously shown that overexpression in Chinese hamster ovary (CHO) cells of ß(1,4)-N-acetylglucosaminyltransferase I11 ("GnTII17y), a glycosyltransferase catalyzing the formation of bisected oligosaccharides, significantly increases the in vitro ADCC activity of an antineuroblastoma chimeric monoclonal antibody (chCE7) produced by the engineered CHO cells. (See Umana, P. et al., Nature Biotechnol. 17: 176-180 (1999); and WO 99/154342, the entire contents of which are hereby incorporated by reference). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17: 176-180 (1999)). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies

The term "expression of the CD20" antigen is intended to indicate an significant level of expression of the CD20 antigen in a cell, preferably on the cell surface of a T- or B- Cell, more preferably a B-cell, from a tumor or cancer, respectively, preferably a non-solid tumor. Patients having a "CD20 expressing cancer" can be determined by standard assays known in the art. "Expression of the CD20" antigen is also preferable intended to indicate an significant level of expression of the CD20 antigen in a cell, preferably on the cell surface of a T- or B- Cell, more preferably a B-cell, in an autoimmune disease. E.g. CD20 antigen expression is measured using immunohistochemical (IHC) detection, FACS or via PCR-based detection of the corresponding mRNA.

The term "CD20 expressing cancer" as used herein refers preferably to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

Preferably the CD20 expressing cancer is a B-Cell Non-Hodgkin's lymphomas (NHL). Especially the CD20 expressing cancer a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

As used herein, "autoimmune disease" relates to a disease or disorder arising from and directed against an individual's own tissues. Examples of autoimmune diseases or disorders include, but are not limited to arthritis (rheumatoid arthritis, juvenile rheumatoid arthritis, osteoarthritis, psoriatic arthritis), psoriasis, dermatitis, polymyositis/dermatomyositis, toxic epidermal necrolysis, systemic scleroderma and sclerosis, responses associated with 15 inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), juvenile onset diabetes, multiple sclerosis, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Bechet disease, Castleman's syndrome, Goodpasture's syndrome, Lambert-Eaton Myasthenic Syndrome, Reynaud's syndrome, Sjorgen's syndrome, Stevens Johnson syndrome, pemphigoid bullous, pemphigus, autoimmune polyendocrinopathies, s nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular I endocrinopathy syndromes), Type I diabetes also referred to as insulin-dependent diabetes i mellitus (IDDM) and Sheehan's syndrome; autoimmune hepatitis, lymphoid interstitial pneumonitis (HIV), bronchiolitis obliterans (non-transplant) vs NSIP, Guillain-Barre' syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, amyotrophic lateral sclerosis (ALS), coronary artery disease etc.

Therapeutic formulations of the antibodies used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed.

The term "surfactant" as used herein denotes a pharmaceutically acceptable surface-active agent. In the formulation of the invention, the amount of surfactant is described a percentage expressed in weight/volume. The most commonly used weight/volume unit is mg/mL. Suitable pharmaceutically acceptable surfactants comprise but are not limited to non-ionic surfactants such as TWEEN™, PLURONICS™ or polyethylene glycol (PEG). Furthermore, comprised but not limited to are polyethylen-sorbitan-fatty acid esters, polyethylene-polypropylene glycols, polyoxyethylene-stearates and sodium dodecyl sulphates. Preferred polyethylen-sorbitan-are polyethylen(20)-sorbitan-esters (synonym to polysorbate 20, sold under the trademark Tween 20™) and polyoxyethylen(20)sorbitanmonooleat (synonym to polysorbate 80 sold under the trademark Tween 80™). Preferred polyethylene-polypropylene glycols are those sold under the names Pluronic® F68 or Poloxamer 188™. Most preferred is Poloxamer 188™. Preferred polyoxyethylene-stearates are those sold under the trademark Myrj™. Preferred Polyoxyethylene monolauryl ether are those sold under the trademark Brij™. When polyethylen-sorbitan-polyethylen(20)-sorbitan-esters (Tween 20™) and polyoxyethylen(20)sorbitanmonooleat (Tween 80™) are used they are generally used in an amount of about 0.001 to about 1%, preferably of about 0.005 to about 0.1% and still preferably about 0.01% to about 0.04%w/v.

The term "buffer" as used herein denotes a pharmaceutically acceptable buffer. Suitable pharmaceutically acceptable buffer comprise but are not limited to histidine-buffers, citrate-buffers, succinate-buffers, acetate-buffers and phosphate-buffers. Preferred buffers comprise L-histidine or mixtures of L-histidine with L-histidine hydrochloride with isotonicity agents and potentially pH adjustment with an acid or a base known in the art. Most preferred is L-histidine. The abovementioned histidine-buffers are generally used in an amount of about 1mM to about 100 mM, preferably of about 5 mM to about 50 mM and still more preferably of about 20 mM. Independently from the buffer used, the pH will be adjusted at a value comprising about 4.5 to about 7.0 and preferably about 5.5 to about 6.5 and still preferably about 6.0 by adjustment with an acid or base known in the art or by using adequate mixtures of buffer components or both.

The term "isotonicity agents" as used herein denotes pharmaceutically acceptable isotonicity agents. Isotonicity agents are used to provide an isotonic formulation. An isotonic formulation is liquid or liquid reconstituted from a solid form, e.g. a lyophilized form and denotes a solution having the same tonicity as some other solution with which it is compared, such as physiologic salt solution and the blood serum. Suitable isotonicity agents comprise but are not limited to salts, including but not limited to sodium chloride(NaCl) or potassium chloride, sugars including but not limited to glucose, sucrose, trehalose or glycerine and any component from the group of amino acids, sugars, salts and combinations thereof. Isotonicity agents are generally used in a total amount of about 5 mM to about 350 mM.

The term "liquid" as used herein in connection with the formulation according to the invention denotes a formulation which is liquid at a temperature of at least about 2 to about 8 °C.

The term "lyophilized" as used herein in connection with the formulation according to the invention denotes a formulation which is dried by freezing the formulation and subsequently subliming the ice from the frozen content by any freeze-drying methods known in the art, for example commercially available freeze-drying devices.

The term "salts" as used herein denotes a salt in an amount of about 1 mM to about 500 mM. Non-limiting examples of salts include salts of any combinations of the cations sodium potassium, calcium or magnesium with anions chloride, phosphate, citrate, succinate, sulphate or mixtures thereof.

The term "amino acid" as used herein denotes an amino acid in an amount of about 1 to about 100 mg/mL comprising but not limited to arginine, glycine, ornithine, glutamine, asparagine, lysine, histidine, glutamic acid, asparagic acid, isoleucine, leucine, alanine, phenylalanine, tyrosine, tryptophane, methionine, serine, proline.

The term "sugar" as used herein denotes a pharmaceutically acceptable sugar used in an amount of about 25 mM to about 500 mM. Preferred is 100 to 300 mM. More preferred is 220 to 260 mM. Most preferred is 240 mM. Suitable sugars comprise but are not limited to monosaccharides and disaccharides. Non-limiting examples of sugars according to the invention include trehalose, sucrose, mannitol, sorbitol, lactose, glucose, mannose, maltose, galactose, fructose, sorbose, raffinose, glucosamine, N-Methylglucosamine (so-called "Meglumine"), galactosamine and neuraminic acid and combinations thereof. Most preferred is trehalose.

The term "stabilizer" refers to pharmaceutically acceptable stabilizers, like for example but not limited to amino acids and sugars as described in the above sections as well as commercially available dextrans of any kind and molecular weight as known in the art.

The term "antioxidant" denotes a pharmaceutically acceptable antioxidant. This may include excipients such as methionine, benzylalcohol or any other excipient used to minimize oxidation.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The formulation according to the invention can be in a liquid form, in a lyophilized form or in a liquid form reconstituted from a lyophilized form.

In one embodiment the formulation according to the invention is a lyophilized formulation. The lyophilized formulation according to the invention has the advantage of an improved stability with regard to the formation of particulates and aggregates of higher molecular weight that is usually difficult to be achieved with liquid formulations at the same concentration of the described anti-CD20 antibody.

The formulation according to the invention can be administered by intravenous (i.v.), subcutaneous (s.c.) or any other parental administration means such as those known in the pharmaceutical art.

Furthermore, a formulation according to the invention may comprise preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; chelating agents such as EDTA; salt-forming counter-ions such as sodium; metal complexes (e.g. Zn-protein complexes).

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

Preferably, the formulation of the invention comprises one or more isotonicity agents in an amount of about 5 mM to about 350 mM as defined hereinabove.

Preferably, the formulation of the invention comprises a sugar in an amount of about 25 mM to about 500 mM as defined hereinabove.

Preferably also, the formulation of the invention further comprises one or more of the following ingredients: antioxidants, ascorbic acid, Glutathion, preservatives, e.g., m-cresol, phenol, benzylalcohol, methylparaben, propylparaben, chlorbutanol, thiomersal, benzalkoniumchloride, polyethylenglycole, e.g. PEG 3000, 3350, 4000, 6000, albumine, human serum albumin (HSA), bovines serum albumin (BSA), polyhydric alcohol, glycerol, ethanol, mannitol, salts, acetate salts (e.g. sodium acetate), magnesiumchloride, calciumchloride, tromethamine, EDTA, (e.g. Na-EDTA).

Preferably also, the formulation of the invention further comprises one or more stabilizers as defined hereinabove and ingredients also known in the art as "lyoprotectants" such as sugars, sugar alcohols, amino acids and dextrans as known in the art.

Described are the following formulations, either in the liquid, lyophilized or liquid reconstituted from lyophilized forms:

> or
> 10mg/mL of
> HuMab<CD20>,
> 0.01% polysorbate 20 w/v,
> 20 mM L-histidine, and
> 140 mM sodium chloride,
> at pH 6.0;

> or
> 15mg/mL of
> HuMab<CD20>,
> Optionally 0.001 to 1% w/v of a surfactant,
> 20 mM L-histidine,
> at pH 6.0;

> or
> 10mg/mL of
> HuMab<CD20>,
> 0.02% polysorbate 20 w/v,
> 20 mM L-histidine, and
> 240 mM trehalose,
> at pH 6.0;

> or
> 25mg/mL of
> HuMab<CD20>,
> 0.02% polysorbate 20 w/v,
> 20 mM L-histidine, and
> 240 mM trehalose,
> at pH 6.0;

> or
> 25mg/mL of
> HuMab<CD20>,
> 0.02% Poloxamer188™ w/v,
> 20 mM L-histidine, and
> 240mM trehalose,
> at pH 6.0;

> or
> 25mg/mL of
> HuMab<CD20>,
> 0.01% Poloxamer188™ w/v,
> 20 mM L-histidine, and
> 240mM trehalose,
> at pH 6.0;

> or
> 25mg/mL of
> HuMab<CD20>,
> 0.1% Poloxamer188™ w/v,

20 mM L-histidine, and
240mM trehalose,
at pH 6.0;

or
25mg/mL of
HuMab<CD20>,
0.02% Polysorbate 80 w/v,
20 mM L-histidine, and
240mM trehalose,
at pH 6.0;

or
25mg/mL of
HuMab<CD20>,
0.1% Polysorbate 80 w/v,
20 mM Acetate, and
240mM trehalose,
at pH 5.5;

or
25mg/mL of
HuMab<CD20>,
0.1% Polysorbate 80 w/v,
20 mM Acetate, and
140mM Sodium chloride,
at pH 5.5;

or
30mg/mL of
HuMab<CD20>,
0.01% Poloxamer188™ w/v,
20 mM L-histidine, and
200mM trehalose,
at pH 6.5;

The formulations of the invention are described in the claims In a preferred embodiment of the formulation according to the invention, the formulation is in a lyophilized form and comprises after reconstitution with the appropriate amount of water for injection:

10mg/mL of
HuMab<CD20>,
0.02% polysorbate 20 w/v,
20 mM L-histidine, and
240 mM trehalose,
at pH 6.0;

This formulation shows a good stability upon storage at 2-8°C and 25° with adequate stability with regard to physical endpoints such as aggregation and chemical endpoints such as fragmentation.

In a preferred embodiment of the formulation according to the invention, the formulation is in a liquid form:

25mg/mL of
HuMab<CD20>,
0.02% Poloxamer188™ w/v,
20 mM L-histidine, and
240mM trehalose,
at pH 6.0;

In a preferred embodiment, the formulation is useful for preventing or reducing metastasis or further dissemination in such a patient suffering from CD20 expressing cancer. The formulation is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the formulation is useful for increasing the response rate in a group of patients.

In the context of this invention, additional other cytotoxic, chemotherapeutic or anti-cancer agents, or compounds that enhance the effects of such agents may be used in combination with the anti-CD20 antibody formulation according to the invention.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thioguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably the anti-CD20 antibody combination treatment is used without such additional agents.

The use of the cytotoxic and anticancer agents described above as well as antiproliferative target-specific anticancer drug like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to the anti-CD20 antibody formulation according to the invention. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. Preferably the anti-CD20 antibody formulation according to the invention is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the formulation according to the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The antibody formulation is administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. Intravenous or subcutaneous administration of the antibodies is preferred.

The invention further comprises a kit characterized in comprising a container, a composition within the container comprising said formulation of the anti-CD20 antibody, and a package insert instructing the user of the formulation to administer said formulation of the anti-CD20 antibody to a patient suffering from CD20 expressing cancer.

The term "package insert" refers to instructions customarily included in commercial packages of therapeutic products, which may include information about the indications, usage, dosage, administration, contraindications and/or warnings concerning the use of such therapeutic products.

In a preferred embodiment, the article of manufacture containers may further include a pharmaceutically acceptable carrier. The article of manufacture may further include a sterile diluent, which is preferably stored in a separate additional container.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

In yet another embodiment of the invention, the formulation according to the invention comprises a type I anti-CD20 antibody which is in that is co-administered with a type II anti-CD20 antibody according to the invention. The formulations according to the invention may be two separate formulations for each of the anti-CD20 antibodies. Alternatively the formulation herein may also contain both antibodies in one formulation.

In yet another embodiment of the invention, the formulation according to the invention comprises a anti-CD20 antibody in that said anti-CD20 antibody is co-administered with an anti-Bcl-2 active agent. The term "Bcl-2" as used herein refers to the Bcl-2 protein(Swiss Prot ID No. P10415), a member of the Bcl-2 family of proteins. The term "anti-Bcl-2 active agent" comprises "anti-Bcl-2 antisense nucleotides" and Bcl-2 inhibitors". The "anti-Bcl-2 antisense nucleotides" down-regulate the Bcl-2 mRNA levels and reduces Bcl-2 protein expression. Examples of such anti-Bcl-2 antisense nucleotides include Oblimersen and SPC-2996. ABT-737 as used herein means N-[4-[4-(4'-Chlorobiphenyl-2-ylmethyl)piperazin-1-yl]benzoyl]-3-[3-(dimethylamino)-1(R)-(phenylsulfanylmethyl)propylamino]-4-nitrobenzenesulfonamide; 4-[4-(4'-Chlorobiphenyl-2-ylmethyl)piperazin-1-yl]-N-[3-[3-(dimethylamino)-1(R)-(phenylsulfanylmethyl)propylamino]-4-nitrophenylsulfonyl]benzamide, a Bcl-2 inhibitor, which is described in WO 2006/099667 or Corey, S., et al., Cancer Cell (2005) 5-6. BT-263 as used herein means a Bcl-2 inhibitor, which is described in US 2007027135. Preferably the anti-Bcl-2 active agent is selected from Oblimersen, SPC-2996, TA-402, Gossypol, AT-101, Obatoclax mesylate, A-371191, A-385358, A-438744, ABT-737, AT-101, BL-11, BL-193, GX-15-003, 2-Methoxyantimycin $A_3$, HA-14-1, KF-67544, Purpurogallin, TP-TW-37, YC-137 and Z-24. referably the anti-Bcl-2 active agent is a Bcl-2 protein binding inhibitor with an IC50 of the anti-Bcl-2 inhibitory activity of 5 μM or less. Such Bcl-2 protein binding inhibitor is preferably selected from Gossypol, AT-101, Obatoclax mesylate, ABT-263 and ABT-737, more preferably from ABT-263 or ABT-737.

In yet another embodiment of the invention, the formulation according to the invention comprises a anti-CD20 antibody in that said anti-CD20 antibody is co-administered with a proteasome inhibitor. The term "proteasome inhibitor" as used herein refers to agents which inhibit the activity of the 26S proteasome. Such proteasome inhibitors include inter alia e.g. peptide derivatives such as peptide aldehydes (e.g. MG132, MG115, CEP-1615, PSI, or immunoproteasome specific inhibitor IPSI-001 (Cbz-LnL-CHO = N-carbobenzyloxy-leucyl-norleucinal, see US 20060241056), peptide boronates (e.g. bortezomib (PS-341) or DFLB), peptide epoxyketones (e.g. epoxomicin, dihydroeponemycin, or epoxomicin derivative carfilzomib (PR-171)), or peptide vinyl sulfones (e.g. NLVS) and non-peptide derivatives such as salinosporamide A (NPI-0052), salinosporamide A derivates, lactacystin or lactacystin derivatives (e.g. clasto-lactacystin-L-lactone (omuralide) or PS-519). The different types and structures of said proteasome inhibitors are described e.g. in Kisselev, A.L., et al., Chem Biol (2001) 739-758, WO 2004/004749 and Joazeiro, C., et al., Res 66(16) (2006) 7840-7842), Kanagasabaphy, et al., Curr Opin Investig Drugs 8 (2007) 447-51, Adams, J., Nat Rev Cancer 4 (2004) 349-360 and US 20060241056.

Preferably such proteasome inhibitor is selected from peptide aldehydes (preferably N-carbobenzyloxy-leucyl-norleucinal (IPSI-001)), peptide boronates (preferably bortezomib (PS-341)), peptide epoxyketones (preferably epoxomicin derivative carfilzomib (PR-171)), or salinosporamide A (NPI-0052). More preferably such proteasome inhibitor is selected from bortezomib (PS-341), carfilzomib (PR-171), salinosporamide A (NPI-0052) or N-carbobenzyloxy-leucyl-norleucinal (IPSI-001).

In a preferred embodiment the proteasome inhibitor is a peptide derivative selected from peptide aldehydes (preferably N-carbobenzyloxy-leucyl-norleucinal (IPSI-001)), peptide boronates (preferably bortezomib (PS-34)) or peptide epoxyketones. In another preferred embodiment the proteasome inhibitor is a peptide boronate (preferably bortezomib (PS-341; see, e.g., Adams, Cur. Opin. Chem Biol. 6 (2002) 493-500 and US 5,780,454)).

Preferably the proteasome inhibitor has an IC50 of the anti-proteasome inhibitory activity of 5 $\mu$M or less, more preferably of 1 $\mu$M or less. A Cell-Based Assay for identifying such proteasome inhibitors and for the determination of the IC50 of the anti-proteasome inhibitory activity (via serial dilutions and calculation using a non-linear curve fit (XLfit software (ID Business Solution Ltd., Guilford, Surrey, UK)) is described in Moravec, et al., Cell Notes 15 (2006) 4-7 using Proteasome-Glo™ Cell-Based Assay Reagent from Promega with U266 cells (human plasma myeloma). This "add-mix-measure" assay measures the chymotrypsin-like protease activity associated with the proteasome in cultured cells.

Besides IPSI-001 (Cbz-LnL-CHO = N-carbobenzyloxy-leucyl-norleucinal) also the following peptide derivatives of US 20060241056 are preferred proteasome inhibitors: N-carbobenzyloxy-homophenylalanyl-phenylalanylal, N-carbobenzyloxy-leucyl-phenylalanylal, N-carbobenzyloxy-alanyl-phenylalanylal, N-carbobenzyloxy-glycyl-prolyl-alanyl-phenylalanylal, N-carbobenzyloxy-glycyl-prolyl-phenylalanyl-phenylalanylal, N-carbobenzyloxy-glycyl-phenylalanyl-phenylalanylal, N-carbobenzyloxy-leucyl-norleucine boronic acid, N-carbobenzyloxy-phenylalanyl-phenylalanine boronic acid, N-carbobenzyloxy-homophenylalanyl-phenylalanine boronic acid, N-carbobenzyloxy-leucyl-phenylalanine boronic acid, N-carbobenzyloxy-glycyl-prolyl-alanylphenylalanine boronic acid, N-carbobenzyloxy-glycyl-prolyl-phenylalanyl-phenylalanine boronic acid, N-carbobenzyloxy-leucyl-leucyl-phenylalanine boronic acid, N-carbobenzyloxy-glycyl-phenylalanyl-phenylalanine boronic acid, N-carbobenzyloxy-leucyl-norleucine methyl vinyl sulfone, N-carbobenzyloxy-phenylalanyl-phenylalanine methyl vinyl sulfone, N-carbobenzyloxy-homophenylalanyl-phenylalanine methyl vinyl sulfone, N-carbobenzyloxy-leucyl-phenytalanine methyl vinyl sulfone, N-carbobenzyloxy-alanyl-phenylalanine methyl vinyl sulfone, N-carbobenzyloxy-glycyl-prolyl-alanyl-phenylalanine methyl vinyl sulfone, N-carbobenzyloxy-glycyl-prolyl-phenylalanyl-phenylalanine methyl vinyl sulfone, N-carbobenzyloxy-leucyl-I$\theta$ucyl-phenylalanine methyl vinyl sulfone, N-carbobenzyloxy-glycyl-phenylalanyl-phenylalanine methyl vinyl sulfone, N-carbobenzyloxy-leucyl-norleucine epoxy ketone, N-carbobenzyloxy-phenylalanyl-phenylalanine epoxy ketone, N-carbobenzyloxy-homophenylalanyl-phenylalanine epoxy ketone, N-carbobenzyloxy-leucyl-phenylalanine epoxy ketone, N-carbobenzyloxy-alanyl-phenylalanine epoxy ketone, N-carbobenzyloxy-glycyl-prolyl-alanyl-phenylalanine epoxy ketone, N-carbobenzyloxy-glycyl-prolyl-phenylalanyl-phenylalanine epoxy ketone, N-carbobenzyloxy-leucyl-leucyl-phenylalanine epoxy ketone, and N-carbobenzyloxy-glycyl-phenylalanyl-phenylalanine epoxy ketone.

The following examples and figures are provided to aid the understanding of the present invention, the true scope of which is set forth in the appended claims. It is understood that modifications can be made in the procedures set forth without departing from the spirit of the invention.

EXAMPLES

Example 1

The following formulations, either in the liquid, lyophilized or liquid reconstituted from lyophilized forms were prepared:

    15mg/mL HuMab<CD20>,
    0.01% polysorbate 20 w/v,
    20 mM L-histidine, and
    140 mM sodium chloride,
    at pH 6.0;

    10mg/mL HuMab<CD20>,
    0.01 % polysorbate 20 w/v,
    20 mM L-histidine, and
    140 mM sodium chloride,
    at pH 6.0;

    15mg/mL HuMab<CD20>,
    20 mM L-histidine,
    at pH 6.0;

    10mg/mL HuMab<CD20>,
    0.02% polysorbate 20 w/v,

20 mM L-histidine, and
240 mM trehalose,
at pH 6.0;

25mg/mL HuMab<CD20>,
0.02% polysorbate 20 w/v,
20 mM L-histidine, and
240 mM trehalose,
at pH 6.0.

Also prepared was a lyophilized form which comprises after reconstitution with the appropriate amount of water for injection:

10mg/mL HuMab<CD20>,
0.02% polysorbate 20 w/v,
20 mM L-histidine, and
240 mM trehalose,
at pH 6.0;

This formulation shows a good stability upon storage at 2-8°C and 25° with adequate stability with regard to physical endpoints such as aggregation and chemical endpoints such as fragmentation.

Liquid and lyophilised drug product formulations for parenteral administration according to the invention were developed as follows:

<u>Preparation of liquid formulations.</u>

Formulations of HuMab<CD20> were prepared by homogenization of solutions of HuMab<CD20> in the production buffer (e.g. 20 mM histidine buffer at pH approx. 6.0 or 20mM histidine buffer at pH approx. 6.0 containing 140mM sodium chloride and 0.01% (w/v) polysorbate 20). Formulations of HuMab<CD20> can also be prepared by adjusting the protein concentration to the desired concentration by dilution with buffer. Excipients for stabilizing the protein and for tonicity adjustment were added as required and can be added in dissolved form or alternatively as solid. Surfactant was added to the formulations as a stock solution as required. All formulations were sterile filtered through 0.22 $\mu$m filters and aseptically aliquoted into sterile glass vials and closed with rubber stoppers and alucrimp caps. These formulations were stored at different temperatures for different intervals of time and removed for analysis at the timepoints indicated in the individual paragraphs. Formulations were analyzed 1) by UV spectrophotometry, 2) by Size Exclusion Chromatography (SEC), 3) for visible and subvisible particles, 4) by Ion exchange chromatography (IEC) and 5) by turbidity of the solution.

<u>Preparation of lyophilised formulations.</u>

Solutions of HuMab<CD20> were either prepared as described above for liquid formulations, or manufactured by homogenizing HuMab<CD20> solutions of HuMab<CD20> in 20mM histidine buffer at pH approx. 6.0, containing a sugar and a surfactant. All formulations were sterile filtered through 0.22 $\mu$m filters and aseptically aliquoted into sterile glass vials. The vials were partly closed with rubber stoppers suitable for the use in lyophilization processes and transferred to the drying chamber of the lyophilizer. Any lyophilisation method known in the art is intended to be within the scope of the invention. For example, the lyophilisation process used for this study included the cooling of the formulation from room temperature to approx 5°C (pre-cooling) followed by a freezing at -40°C (Freeze I) at a ramping rate of about 1°C/min to 5°C/min. The first drying step can take place at a ramping rate of 0.3 to 0.5°C / min from -40°C to -30°C and then hold at -30°C for at least 50 hours at a chamber pressure of approx. 75 to 80 mTorr. A second drying step can take place at a ramping rate of 0.1 to 0.3°C / min from -30°C to 25°C and hold at 25°C for at least 5 hours at a chamber pressure of about 50 to 80 mTorr (the applied drying schedule is provided in Table 1). HuMab<CD20> formulations which were dried using the described lyophilisation processes were found to have conveniently quick reconstitution times of about 2-3 minutes. All lyophilised cakes in this study had a residual water content of approximately 0.1 to 1.0% as determined by Karl-Fischer method. The lyophilised vials were stored at different temperatures for different intervals of time. The lyophilised formulations were reconstituted with the respective volume of water for injection (WFI) prior to 1) analysis by UV spectrophotometry, 2) determination of the reconstitution time, 3) analysis by Size Exclusion Chromatography (SEC) 4) by Ion exchange chromatography (IEC), 5) determination of subvisible and visible particles and 6) by turbidity of the solution.

Size exclusion chromatography (SEC) was performed to detect soluble high molecular weight species (aggregates) and low molecular weight hydrolysis products in the formulations. The method used a suitable HPLC instrument equipped with a UV detector (detection wavelength 280 nm) and a Zorbax GF-250 column ( 9.4 x 250 mm, Agilent); the method used 200mM sodium phosphate pH 7.0 as mobile phase.

Ion Exchange Chromatography (IEC) was performed to detect chemical degradation products altering the net charge of HuMab<CD20> in the formulations. The method used a suitable HPLC instrument equipped with a UV detector (detection wavelength 220 and 280nm) and a Dionex ProPac WCX-10 column ( 4mm x 250mm). 10mM sodium phosphate buffer pH 6.0 in $H_2O$ and 10mM sodium phosphate buffer pH 6.0 + 0.75M NaCl were used as mobile phases A and B, respectively, with a flow rate of 1.0mL/min.

The UV spectroscopy for determination of the protein concentration was performed on a Varian Cary Bio UV spectrophotometer at 280 nm.

For the determination of the turbidity, opalescence was measured in FTU (turbidity units) using a HACH 2100AN turbidimeter at room temperature.

Samples were analyzed for subvisible particles by using a HIAC Royco PharmaSpec (HRLD-150), and for visible particles by using a Seidenader V90-T visual inspection instrument.

Table 1 Freeze-drying Cycle

| Step | Shelf temperature (°C) | Ramp Rate (°C/min) | Hold time (min) | Vacuum Set point (mTorr) |
|---|---|---|---|---|
| Pre-cooling | 5°C | 0.0 | 60 | - |
| Freeze I | -40°C | 1.0 | 120 | - |
| Prim Drying | -30°C | 0.5 | 3720 | 80 |
| Sec Drying | +25°C | 0.2 | 300 | 80 |

**Stability data of liquid HuMab<CD20> drug product formulations according to this invention**

Formulation 15mg/mL HuMab<CD20>, 20 mM L-histidine, pH 6.0,

| Storage cond. | Storage Time (months) | Protein (mg/mL) | SEC Monomer (%) | SEC HMW (%) | Turbidity | IEC Significant Changes | Visible particles | Subvisible particles |
|---|---|---|---|---|---|---|---|---|
| Initial | | 14.5 | 98.4 | 1.6 | 5.9 | No | Pass | Pass |
| 2-8°C | 1 | 14.7 | 98.1 | 1.8 | 5.9 | No | Pass | Pass |
| | 3 | 15.5 | 97.9 | 2.0 | 6.1 | No | Pass | Pass |
| | 6 | 14.2 | 97.7 | 2.1 | 5.7 | No | Pass | Pass |
| 25°C | 1 | 14.8 | 98.0 | 1.7 | 5.7 | No | Pass | Pass |
| | 3 | 15.0 | 97.8 | 1.8 | 5.8 | No | Pass | Pass |
| | 6 | 14.6 | 97.3 | 1.9 | 6.1 | Yes | Fail | Pass |
| 40°C | 1 | 14.8 | 97.6 | 1.6 | 5.8 | No | Pass | Pass |
| | 3 | 15.1 | 96.1 | 1.9 | 6.2 | Yes | Pass | Pass |
| -20°C | 1 | 14.8 | 98.4 | 1.6 | 6.3 | n/d | Fail | Pass |
| | 3 | 15.1 | 98.1 | 1.8 | 6.2 | No | Fail | Pass |
| | 6 | 14.3 | 97.7 | 2.3 | 6.1 | No | Fail | Pass |
| -80°C | 3 | 15.0 | 98.2 | 1.6 | 5.8 | No | Fail | Pass |
| | 6 | 14.9 | 98.4 | 1.6 | 5.7 | No | Fail | Pass |
| n/d: not determined<br>Pass: free to essentially free of visible particles, max 6000 particles $\geq 10\mu m$ /container, max 600 particles $\geq 25\mu m$ /container | | | | | | | | |

Formulation 10mg/mL HuMab<CD20>, 20 mM L-histidine, 240mM trehalose, 0.02% w/v Polysorbate 20, pH 6.0,

| Storage con d. | Storage Time (months) | Protein (mg/mL) | SEC Monomer (%) | SEC HMW (%) | Turbidity | IEC Significant Changes | Visible particles | Subvisible particles |
|---|---|---|---|---|---|---|---|---|
| Initial | | 11.0 | 98.4 | 1.6 | 3.9 | No | Pass | Pass |
| 2-8°C | 1 | 11.1 | 98.3 | 1.6 | 4.0 | No | Fail | Pass |
| | 3 | 11.3 | 98.2 | 1.7 | 6.6 | No | Fail | Pass |
| | 6 | 10.8 | 98.1 | 1.7 | 8.3 | No | Fail | Pass |
| 25°C | 1 | 11.0 | 98.3 | 1.4 | 6.4 | No | Fail | Pass |
| | 3 | 11.3 | 98.2 | 1.5 | 6.7 | No | Fail | Pass |
| | 6 | 11.2 | 98.8 | 1.4 | 6.7 | Yes | Fail | Pass |
| 40°C | 1 | 11.1 | 98.0 | 1.3 | 7.5 | No | Fail | Pass |
| | 3 | 11.2 | 96.7 | 1.5 | 4.9 | Yes | Fail | Pass |
| -20°C | 1 | 11.0 | 98.5 | 1.5 | 3.4 | No | Pass | Pass |
| | 3 | 11.2 | 98.3 | 1.6 | 4.0 | No | Pass | Pass |
| | 6 | n/d | n/d | n/d | n/d | n/d | n/d | n/d |
| -80°C | 3 | 11.2 | 98.3 | 1.6 | 3.7 | No | Pass | Pass |
| | 6 | 11.0 | 98.5 | 1.5 | 3.8 | No | Pass | Pass |
| n/d = not determined  Pass: free to essentially free of visible particles, max 6000 particles $\geq 10\mu m$ /container, max 600 particles $\geq 25\mu m$ /container | | | | | | | | |

Stability data of lyophilized huMAb<CD20> drug product formulations according to this invention

Formulation 10mg/mL HuMab<CD20>, 20 mM L-histidine, 240mM trehalose, 0.02% w/v Polysorbate 20, pH 6.0,

| Storage cond. | Storage Time (months) | Protein (mg/mL) | SEC Monomer (%) | SEC HMW (%) | Turbidity | IEC Main peak (%) | Visible particles | Subvisible particles |
|---|---|---|---|---|---|---|---|---|
| Initial | | 10.4 | 98.9 | 1.1 | 2.9 | 54.8 | Pass | Pass |
| **2-8°C** | 1 | 10.5 | 98.9 | 1.1 | 2.9 | n/d | Pass | Pass |
| | 3 | 10.4 | 98.9 | 1.1 | 2.9 | n/d | Pass | Pass |
| | 6 | 10.4 | 98.9 | 1.1 | 3.4 | 53.1 | Pass | Pass |
| | 12 | 10.4 | 98.9 | 1.1 | 3.1 | 56.6 | Pass | Pass |
| | 24 | 10.3 | 98.9 | 1.1 | 3.1 | 55.6 | Pass | Pass |
| **25°C** | 1 | 10.2 | 98.9 | 1.1 | 2.9 | n/d | Pass | Pass |
| | 3 | 105 | 98.9 | 1.1 | 2.9 | n/d | Pass | Pass |
| | 6 | 10.4 | 98.9 | 1.1 | 3.3 | 52.9 | Pass | Pass |
| | 12 | 10.4 | 98.8 | 1.2 | 3.1 | 56.9 | Pass | Pass |
| **40°C** | 1 | 10.5 | 98.9 | 1.1 | 3.0 | n/d | Pass | Pass |
| | 3 | 10.4 | 98.9 | 1.1 | 3.0 | n/d | Pass | Pass |
| | 6 | 10.5 | 98.9 | 1.1 | 3.4 | 50.9 | Pass | Pass |
| n/d = not determined Pass: free to essentially free of visible particles, max 6000 particles $\geq 10\mu m$ /container, max 600 particles $\geq 25\mu m$ /container | | | | | | | | |

Example 2

The following formulations, either in the liquid, lyophilized or liquid reconstituted from lyophilized forms were prepared:

25mg/mL HuMab<CD20>,
0.02% Poloxamer188™ w/v,
20 mM L-histidine, and
240mM trehalose,
at pH 6.0;

or
25mg/mL HuMab<CD20>,
0.01% Poloxamer188™ w/v,
20 mM L-histidine, and
240mM trehalose,
at pH 6.0;

or
25mg/mL HuMab<CD20>,
0.1% Poloxamer188™ w/v,
20 mM L-histidine, and
240mM trehalose,
at pH 6.0;

or
25mg/mL HuMab<CD20>,
0.02% Polysorbate 80 w/v,
20 mM L-histidine, and
240mM trehalose,
at pH 6.0;

or
25mg/mL HuMab<CD20>,
0.1% Polysorbate 80 w/v,
20 mM Acetate, and
240mM trehalose,
at pH 5.5;

or
25mg/mL HuMab<CD20>,
0.1 % Polysorbate 80 w/v,
20 mM Acetate, and
140mM Sodium chloride,
at pH 5.5;

or
30mg/mL HuMab<CD20>,
0.01% Poloxamer188™ w/v,
20 mM L-histidine, and
200mM trehalose,
at pH 6.5;

Liquid and lyophilised drug product formulations for parenteral administration were prepared as follows:

Preparation of liquid formulations.

Formulations of HuMab<CD20> were prepared by homogenization of solutions of HuMab<CD20> in the production buffer (e.g. 20 mM histidine buffer at pH approx. 6.0 containing 240mM trehalose and 0.02% (w/v) Poloxamer188™). Formulations of HuMab<CD20> can also be prepared by diafiltrating solutions of approx. 10-40 mg/ml HuMab<CD20> in the production buffer (e.g. 20 mM histidine buffer at pH approx. 6.0) by tangential flow filtration (TFF) to increase the protein concentration above target protein concentration and to exchange buffer. Formulations of HuMab<CD20> can also be prepared by adjusting the protein concentration to the desired concentration by dilution with buffer. Excipients for stabilizing the protein and for tonicity adjustment can be added in dissolved form or alternatively as solid. Surfactant was added to the formulations as a stock solution as required. All formulations were sterile filtered through 0.22 μm filters and aseptically aliquoted into sterile glass vials and closed with rubber stoppers and alucrimp caps. These formulations were stored at different temperatures for different intervals of time and removed for analysis at the timepoints indicated in the individual paragraphs. Formulations were analyzed 1) by UV spectrophotometry, 2) by Size Exclusion Chromatography (SEC), 3) for visible and subvisible particles, 4) by Ion exchange chromatography (IEC) and 5) by turbidity of the solution.

Preparation of lyophilised formulations.

Solutions of <CD20> were prepared as described above for liquid formulations. All formulations were sterile filtered through 0.22 μm filters and aseptically aliquoted into sterile glass vials. The vials were partly closed with rubber stoppers suitable for the use in lyophilization processes and transferred to the drying chamber of the lyophilizer. Any lyophilisation method known in the art is intended to be within the scope of the invention. For example, the lyophilisation process used for this study included the cooling of the formulation from room temperature to approx 5°C (pre-cooling) followed by a freezing at -40°C (Freeze I) at a ramping rate of about 1°C/min to 5°C/min. The first drying step can take place at a ramping rate of 0.3 to 0.5°C / min from -40°C to -30°C and then hold at -30°C for at least 50 hours at a chamber pressure of approx. 75 to 80 mTorr. A second drying step can take place at a ramping rate of 0.1 to 0.3°C / min from -30°C to 25°C and hold at 25°C for at least 5 hours at a chamber pressure of about 50 to 80 mTorr (the applied drying schedule is provided in Table 1). HuMab<CD20> formulations which were dried using the described lyophilisation processes were found to have a residual water content of approximately 0.1 to 1.0% as determined by Karl-Fischer method. The lyophilised vials were stored at different temperatures for different intervals of time. The lyophilised formulations were reconstituted with the respective volume of water for injection (WFI) prior to 1) analysis by UV spectrophotometry, 2) analysis by Size Exclusion Chromatography (SEC) 3) by Ion exchange chromatography (IEC), 4) determination of subvisible and visible particles and 5) by turbidity of the solution.

Size exclusion chromatography (SEC) was performed to detect soluble high molecular weight species (aggregates) and low molecular weight hydrolysis products in the formulations. The method used a suitable HPLC instrument equipped with a UV detector (detection wavelength 280 nm) and either a Zorbax GF-250 column ( 9.4 x 250 mm, Agilent) or a TSKgel G3000 SWXL (7.8x300mm); the method used either 200mM sodium phosphate pH 7.0 or 250mM potassium chloride in 200mM potassium phosphate pH 7.0 as mobile phase.

Ion Exchange Chromatography (IEC) was performed to detect chemical degradation products altering the net charge of HuMab<CD20> in the formulations. The method used a suitable HPLC instrument equipped with a UV detector (detection wavelength 220 and 280nm) and a Dionex ProPac WCX-10 column ( 4mm x 250mm). 10mM sodium phosphate buffer pH 6.0 in $H_2O$ and 10mM sodium phosphate buffer pH 6.0 + 0.75M NaCl were used as mobile phases A and B, respectively, with a flow rate of 1.0mL/min.

The UV spectroscopy for determination of the protein concentration was performed on a Varian Cary Bio or a Perkin Elmer UV spectrophotometer at 280 nm.

For the determination of the turbidity, opalescence was measured in FTU (turbidity units) using a HACH 2100AN turbidimeter at room temperature.

Samples were analyzed for subvisible particles by using a HIAC Royco PharmaSpec (HRLD-150), and for visible particles by using a Seidenader V90-T visual inspection instrument.

Table 1 Freeze-drying Cycle

| Step | Shelf temperature (°C) | Ramp Rate (°C/min) | Hold time (min) | Vacuum Set point (mTorr) |
|---|---|---|---|---|
| Pre-cooling | 5°C | 0.0 | 60 | - |
| Freeze I | -40°C | 1.0 | 120 | - |
| Prim Drying | -30°C | 0.5 | 3720 | 80 |
| Sec Drying | +25°C | 0.2 | 300 | 80 |

**Stability data of liquid HuMab<CD20> drug product formulations**

Formulation 25mg/mL HuMab<CD20>, 20mM L-histidine, 240mM trehalose, 0.02% w/v Poloxamer188™, pH 6.0,

| Storage cond. | Storage Time (months) | Protein (mg/mL) | SEC Monomer (%) | SEC HMW (%) | Turbi -dity | IEC Main peak (%) | Visible particles | Subvisible particles |
|---|---|---|---|---|---|---|---|---|
| Initial | | 25.7 | 98.7 | 1.3 | 6.4 | 61.6 | Pass | Pass |
| 2-8°C | 1 | 25.6 | 98.7 | 1.3 | 6.0 | 62.0 | Pass | Pass |
| | 3 | 26.1 | 98.6 | 1.3 | 5.9 | 61.0 | Pass | Pass |
| 25°C | 1 | 25.7 | 98.5 | 1.4 | 6.5 | 62.0 | Pass | Pass |
| | 3 | 26.0 | 97.9 | 1.5 | 5.9 | 58.5 | Pass | Pass |
| 40°C | 1 | 25.7 | 97.9 | 1.6 | 6.6 | 52.6 | Pass | Pass |
| | 3 | 25.7 | 91.0 | 2.4 | 6.7 | 33.6 | Pass | Pass |
| -80°C | 3 | 25.6 | 98.7 | 1.3 | 6.2 | 60.9 | Pass | Pass |
| -20°C | 3 | 25.5 | 98.7 | 1.3 | 6.3 | 60.8 | Pass | Pass |
| Pass: free to essentially free of visible particles, max 6000 particles $\geq 10 \mu m$ /container, max 600 particles $\geq 25 \mu m$ /container | | | | | | | | |

Formulation 25mg/mL HuMab<CD20>, 20mM L-histidine, 240mM trehalose, 0.01% w/v Poloxamer188™, pH 6.0,

| Storage cond. | Storage Time (months) | Protein (mg/mL) | SEC Monomer (%) | SEC HMW (%) | Turbidity | IEC Main peak (%) | Visible particles | Subvisible particles |
|---|---|---|---|---|---|---|---|---|
| Initial | | 26.4 | 99.6 | 0.4 | 5.5 | 72.2 | Pass | Pass |
| 2-8°C | 1 | 26.4 | 99.7 | 0.3 | 5.7 | n/a | Pass | Pass |
| | 2 | 26.3 | 99.5 | 0.5 | 5.7 | 72.0 | Pass | Pass |
| | 3 | 26.3 | 99.5 | 0.5 | 6.3 | 72.6 | Pass | Pass |
| | 9 | 26.2 | 99.4 | 0.6 | 6.0 | 70.8 | Pass | Pass |
| | 12 | 26.4 | 99.5 | 0.5 | 6.5 | 70.1 | Pass | Pass |
| 25°C | 1 | n/a | 99.6 | 0.4 | 5.2 | n/a | Pass | Pass |
| | 2 | n/a | 99.4 | 0.6 | 5.7 | 70.6 | Pass | Pass |
| | 3 | n/a | 99.4 | 0.6 | 6.1 | 66.5 | Pass | Pass |
| | 9 | 26.4 | 95.7 | 0.8 | 5.9 | 58.2 | Pass | Pass |
| | 12 | 26.5 | 95.5 | 0.7 | 6.1 | 54.9 | Pass | Pass |
| 40°C | 1 | n/a | 98.2 | 0.3 | 5.4 | 58.6 | Pass | Pass |
| | 2 | n/a | 97.4 | 0.8 | 5.7 | 50.4 | Pass | Pass |
| | 3 | n/a | 96.7 | 1.0 | 6.3 | n/a | Pass | Pass |
| -80°C | 9 | 25.7 | 99.5 | 0.5 | 5.9 | 72.3 | Pass | Pass |

n/a: not analyzed
Pass: free to essentially free of visible particles, max 6000 particles ≥10μm /container, max 600 particles ≥25μm /container

Formulation 25mg/mL HuMab<CD20>, 20mM L-histidine, 240mM trehalose, 0.1% w/v Poloxamer188™, pH 6.0,

| Storage cond. | Storage Time (months) | Protein (mg/mL) | SEC Monomer (%) | SEC HMW (%) | Turbidity | IEC Main peak (%) | Visible particles | Subvisible particles |
|---|---|---|---|---|---|---|---|---|
| Initial | | 26.4 | 99.6 | 0.4 | 5.5 | 71.5 | Pass | Pass |
| 2-8°C | 1 | 26.7 | 99.7 | 0.3 | 6.2 | n/a | Pass | Pass |
| | 2 | 26.5 | 99.5 | 0.5 | 5.4 | 73.0 | Pass | Pass |
| | 3 | 26.4 | 99.5 | 0.5 | 6.3 | 70.9 | Pass | Pass |
| | 9 | 26.5 | 99.4 | 0.6 | 6.4 | 71.8 | Pass | Pass |
| | 12 | 26.6 | 99.5 | 0.5 | 5.8 | 70.0 | Pass | Pass |
| 25°C | 1 | n/a | 99.6 | 0.4 | 5.2 | n/a | Pass | Pass |
| | 2 | n/a | 99.4 | 0.6 | 5.7 | 70.7 | Pass | Pass |
| | 3 | n/a | 99.4 | 0.6 | 6.2 | 67.4 | Pass | Pass |
| | 9 | 26.6 | 95.7 | 0.8 | 6.2 | 58.6 | Pass | Pass |
| | 12 | 26.6 | 95.4 | 0.7 | 6.0 | 54.9 | Pass | Pass |

(continued)

| Storage cond. | Storage Time (months) | Protein (mg/mL) | SEC Monomer (%) | SEC HMW (%) | Turbi dity | IEC Main peak (%) | Visible particles | Subvisible particles |
|---|---|---|---|---|---|---|---|---|
| 40°C | 1 | n/a | 98.0 | 0.5 | 5.3 | 59.3 | Pass | Pass |
| | 2 | n/a | 97.4 | 0.8 | 5.8 | 51.8 | Pass | Pass |
| | 3 | n/a | 96.6 | 1.1 | 7.0 | n/a | Pass | Pass |
| -80°C | 9 | 26.1 | 99.5 | 0.5 | 5.9 | 71.0 | Pass | Pass |

n/a: not analyzed
Pass: free to essentially free of visible particles, max 6000 particles $\geq 10\mu m$ /container, max 600 particles $\geq 25\mu m$ /container

Formulation 25mg/mL HuMab<CD20>, 20mM Acetate, 240mM trehalose, 0.1% w/v Polysorbate 80, pH 5.5,

| Storage cond. | Storage Time (months) | Protein (mg/mL) | SEC Monomer (%) | SEC HMW (%) | Turbi dity | IEC Main peak (%) | Visible particles | Subvisible particles |
|---|---|---|---|---|---|---|---|---|
| Initial | | 24.6 | 99.6 | 0.4 | 8.2 | 71.9 | Pass | Pass |
| 2-8°C | 1 | 24.8 | 99.7 | 0.3 | 4.9 | n/a | Pass | Pass |
| | 2 | 24.5 | 99.4 | 0.6 | 5.2 | 72.6 | Pass | Pass |
| | 3 | 24.3 | 99.4 | 0.6 | 7.0 | 66.2 | Pass | Pass |
| 25°C | 1 | n/a | 99.6 | 0.4 | 6.7 | n/a | Pass | Pass |
| | 2 | n/a | 99.3 | 0.7 | 7.0 | 71.9 | Pass | Pass |
| | 3 | n/a | 99.2 | 0.8 | 7.3 | 65.1 | Pass | Pass |
| 40°C | 1 | n/a | 97.5 | 1.0 | 26.8 | 52.9 | Pass | Pass |
| | 2 | n/a | 96.0 | 2.1 | 27.8 | 42.1 | Pass | Pass |
| | 3 | n/a | 94.4 | 3.2 | 26.8 | n/a | Pass | Pass |

n/a: not analyzed
Pass: free to essentially free of visible particles, max 6000 particles $\geq 10\mu m$ /container, max 600 particles $\geq 25\mu m$ /container

Formulation 25mg/mL HuMab<CD20>, 20mM Acetate, 140mM sodium chloride, 0.1% w/v Polysorbate 80, pH 5.5,

| Storage cond. | Storage Time (months) | Protein (mg/mL) | SEC Monome r (%) | SEC HMW (%) | Turbi dity | IEC Main peak (%) | Visible particles | Subvisible particles |
|---|---|---|---|---|---|---|---|---|
| Initial | | 23.7 | 99.5 | 0.5 | 11.4 | 70.9 | Pass | Pass |
| 2-8°C | 1 | 24.4 | 99.6 | 0.4 | 11.4 | n/a | Pass | Pass |
| | 2 | 24.2 | 99.4 | 0.6 | 10.5 | 71.5 | Pass | Pass |
| | 3 | 24.2 | 99.3 | 0.7 | 12.5 | 71.3 | Pass | Pass |
| 25°C | 1 | n/a | 99.5 | 0.5 | 12.0 | n/a | Pass | Pass |
| | 2 | n/a | 99.1 | 0.9 | 013.0 | 68.2 | Pass | Pass |

(continued)

| Storage cond. | Storage Time (months) | Protein (mg/mL) | SEC Monomer (%) | SEC HMW (%) | Turbidity | IEC Main peak (%) | Visible particles | Subvisible particles |
|---|---|---|---|---|---|---|---|---|
|  | 3 | n/a | 99.1 | 0.9 | 13.2 | 64.6 | Pass | Pass |
| 40°C | 1 | n/a | 96.9 | 1.2 | 26.8 | 54.0 | Pass | Pass |
|  | 2 | n/a | 95.0 | 2.9 | 26.5 | 46.1 | Pass | Pass |
|  | 3 | n/a | 93.1 | 4.2 | 26.1 | n/a | Pass | Pass |
| n/a: not analyzed<br>Pass: free to essentially free of visible particles, max 6000 particles $\geq 10\mu m$ /container, max 600 particles $\geq 25\mu m$ /container | | | | | | | | |

Formulation 30mg/mL HuMab<CD20>, 20mM L-histidine, 200mM trehalose, 0.01% w/v Poloxamer188™, pH 6.5,

| Storage cond. | Storage Time (months) | Protein (mg/mL) | SEC Monomer (%) | SEC HMW (%) | Turbidity | IEC Main peak (%) | Visible particles | Subvisible particles |
|---|---|---|---|---|---|---|---|---|
| Initial |  | 32.0 | 98.4 | 1.6 | 6.4 | 61.0 | Pass | Pass |
| 2-8°C | 1 | 32.2 | 98.3 | 1.6 | 6.6 | 62.3 | Pass | Pass |
|  | 3 | 32.9 | 98.1 | 1.7 | 5.7 | 60.5 | Pass | Pass |
| 25°C | 1 | 32.2 | 98.1 | 1.8 | 6.2 | 60.9 | Pass | Pass |
|  | 3 | 32.1 | 97.6 | 2.0 | 6.2 | 56.5 | Pass | Pass |
| 40°C | 1 | 32.0 | 97.3 | 2.1 | 6.3 | 50.9 | Pass | Pass |
|  | 3 | 32.3 | 89.9 | 3.0 | 7.2 | 31.1 | Pass | Pass |
| -80°C | 3 | 31.8 | 98.3 | 1.6 | 6.2 | 61.0 | Pass | Pass |
| -20°C | 3 | 32.1 | 98.3 | 1.6 | 6.6 | 60.7 | Pass | Pass |
| Pass: free to essentially free of visible particles, max 6000 particles $\geq 10\mu m$ /container, max 600 particles $\geq 25\mu m$ /container | | | | | | | | |

**Stability data of lyophilized huMAb<CD20> drug product formulations**

Formulation 25mg/mL HuMab<CD20>, 20 mM L-histidine, 240mM trehalose, 0.02% w/v Poloxamer188™

| Storage cond. | Storage Time (months) | Protein (mg/mL) | SEC Monomer (%) | SEC HMW (%) | Turbidity | IEC Main peak (%) | Visible particles | Sub-visible particles |
|---|---|---|---|---|---|---|---|---|
| Initial |  | 25.5 | 98.9 | 1.0 | 6.2 | 61.2 | Pass | Pass |
| 2-8°C | 1 | 25.2 | 99.0 | 1.0 | 5.8 | 63.1 | Pass | Pass |
|  | 3 | 25.3 | 99.0 | 1.0 | 6.1 | 60.4 | Pass | Pass |
| 25°C | 1 | 25.4 | 98.9 | 1.0 | 6.0 | 62.9 | Pass | Pass |
|  | 3 | 25.6 | 98.5 | 1.1 | 6.1 | 60.2 | Pass | Pass |
| 40°C | 1 | 25.3 | 98.9 | 1.1 | 6.3 | 60.6 | Pass | Pass |

(continued)

| Storage cond. | Storage Time (months) | Protein (mg/mL) | SEC Monomer (%) | SEC HMW (%) | Turbi dity | IEC Main peak (%) | Visible particles | Sub-visible particles |
|---|---|---|---|---|---|---|---|---|
| | 3 | 25.9 | 98.5 | 1.3 | 5.9 | 56.9 | Pass | Pass |
| -80°C | 3 | 25.3 | 98.9 | 1.0 | 6.3 | 61.2 | Pass | Pass |
| -20°C | 3 | 25.4 | 98.9 | 1.0 | 6.7 | 60.6 | Pass | Pass |
| Pass: free to essentially free of visible particles, max 6000 particles ≥10μm /container, max 600 particles ≥25μm /container | | | | | | | | |

Formulation 25mg/mL HuMab<CD20>, 20 mM L-histidine, 240mM trehalose, 0.02% w/v Polysorbate 80, pH 6.0,

| Storage cond. | Storage Time (months) | Protein (mg/mL) | SEC Monome r(%) | SEC HMW (%) | Turbi dity | IEC Main peak (%) | Visible particles | Subvisible particles |
|---|---|---|---|---|---|---|---|---|
| Initial | | 27.2 | 99.6 | 0.4 | 6.0 | 72.3 | Pass | Pass |
| 2-8°C | 1 | 27.1 | 99.7 | 0.3 | 6.1 | n/a | Pass | Pass |
| | 2 | 27.0 | 99.5 | 0.5 | 5.8 | 74.0 | Pass | Pass |
| | 3 | 27.0 | 99.5 | 0.5 | 6.2 | 69.8 | Pass | Pass |
| 25°C | 1 | n/a | 99.7 | 0.4 | 6.2 | n/a | Pass | Pass |
| | 2 | n/a | 99.5 | 0.5 | 5.4 | 66.2 | Pass | Pass |
| | 3 | n/a | 99.5 | 0.5 | 5.9 | 68.6 | Pass | Pass |
| 40°C | 1 | n/a | 99.6 | 0.4 | 5.4 | 70.6 | Pass | Pass |
| | 2 | n/a | 99.4 | 0.6 | 5.4 | 70.1 | Pass | Pass |
| | 3 | n/a | 99.3 | 0.7 | 6.3 | n/a | Pass | Pass |
| n/a: not analyzed Pass: free to essentially free of visible particles, max 6000 particles ≥10μm /container, max 600 particles ≥25μm /container | | | | | | | | |

## Claims

1. A formulation, wherein it is in a lyophilized form and comprises:

   10mg/mL of the humanized B-Ly 1 antibody comprising VH B-HH6 and VL B-KV1 of WO 2005/044859,
   0.02% polysorbate 20 w/v,
   20 mM L-histidine, and
   240 mM trehalose,
   at pH 6.0.

2. A formulation, wherein it comprises:

   25mg/mL of the humanized B-Ly 1 antibody comprising VH B-HH6 and VL B-KV1 of WO 2005/044859,
   0.02% Poloxamer188™ w/v,
   20 mM L-histidine, and
   240mM trehalose,
   at pH 6.0;

or
25mg/mL of the humanized B-Ly 1 antibody comprising VH B-HH6 and VL B-KV1 of WO 2005/044859,
0.01% Poloxamer188™ w/v,
20 mM L-histidine, and
240mM trehalose,
at pH 6.0;
or
25mg/mL of the humanized B-Ly 1 antibody comprising VH B-HH6 and VL B-KV1 of WO 2005/044859,
0.1% Poloxamer188™ w/v,
20 mM L-histidine, and
240mM trehalose,
at pH 6.0;
or
25mg/mL of the humanized B-Ly 1 antibody comprising VH B-HH6 and VL B-KV1 of WO 2005/044859,
0.02% Polysorbate 80 w/v,
20 mM L-histidine, and
240mM trehalose,
at pH 6.0;
or
30mg/mL of the humanized B-Ly antibody comprising VH B-HH6 abnd VL B-KV1 of WO 2005/044859,
0.01% Poloxamer188™ w/v,
20 mM L-histidine, and
200mM trehalose,
at pH 6.5.

3. The formulation according to claim 2, wherein it is in a liquid form and comprises:

   25mg/mL of the humanized B-Ly 1 antibody comprising VH B-HH6 and VL B-KV1 of WO 2005/044859,
   0.02% Poloxamer188™ w/v,
   20 mM L-histidine, and
   240mM trehalose,
   at pH 6.0.

4. Use of a formulation according to any one of claims 1 to 3 for the preparation of a medicament useful for treating CD20-related diseases.

5. The use according to claim 4 wherein the disease is selected from the group consisting of B-Cell Non-Hodgkin's lymphomas (NHL), Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia and primary CNS lymphoma.

**Patentansprüche**

1. Formulierung, wobei diese in einer lyophilisierten Form ist und umfasst:

   10 mg/ml des humanisierten B-Ly1-Antikörpers, umfassend VH B-HH6 und VL B-KV1 von WO 2005/044859,
   0.02% Polysorbat 20 w/v,
   20 mM L-Histidin, und
   240 mM Trehalose,
   bei einem pH-Wert von 6.0.

2. Formulierung, wobei diese umfasst:

   25 mg/ml des humanisierten B-Ly1-Antikörpers, umfassend VH B-HH6 und VL B-KV1 von WO 2005/044859,
   0.02% Poloxamer188™ w/v,
   20 mM L-Histidin, und

240 mM Trehalose,
bei einem pH-Wert von 6.0;
oder
25 mg/ml des humanisierten B-Ly1-Antikörpers, umfassend VH B-HH6 und VL B-KV1 von WO 2005/044859,
0.01 % Poloxamer188™ w/v,
20 mM L-Histidin, und
240 mM Trehalose,
bei einem pH-Wert von 6.0;
oder
25 mg/ml des humanisierten B-Ly1-Antikörpers, umfassend VH B-HH6 und VL B-KV1 von WO 2005/044859,
0. 1 % Poloxamer188™ w/v,
20 mM L-Histidin, und
240 mM Trehalose,
bei einem pH-Wert von 6.0;
oder
25 mg/ml des humanisierten B-Ly1-Antikörpers, umfassend VH B-HH6 und VL B-KV1 von WO 2005/044859,
0.02% Polysorbat 80 w/v,
20 mM L-Histidin, und
240 mM Trehalose,
bei einem pH-Wert von 6.0;
oder
30 mg/ml des humanisierten B-Ly1-Antikörpers, umfassend VH B-HH6 und VL B-KV1 von WO 2005/044859,
0.01% Poloxamer188™ w/v,
20 mM L-Histidin, und
200 mM Trehalose,
bei einem pH-Wert von 6.5.

3. Formulierung gemäß Anspruch 2, wobei diese in einer flüssigen Form ist und umfasst:

25 mg/ml des humanisierten B-Ly1-Antikörpers, umfassend VH B-HH6 und VL B-KV1 von WO 2005/044859,
0.02% Poloxamer188™ w/v,
20 mM L-Histidin, und
240 mM Trehalose, bei einem pH-Wert von 6.0.

4. Verwendung der Formulierung gemäß einem der Ansprüche 1 bis 3 für die Herstellung eines Medikaments, das für die Behandlung von mit CD20 in Beziehung stehende Krankheiten geeignet ist.

5. Verwendung nach Anspruch 4, wobei die Krankheit ausgewählt ist aus der Gruppe bestehend aus B-Zell-Non-Hodgkin-Lymphome (NHL), Mantelzelllymphom (MCL), akuter lymphatischer Leukämie (ALL), chronischer lymphatischer Leukämie (CLL), diffusem großzelligem B-Zell-Lymphom (DLCL), Burkitt-Lymphom, Haarzellenleukämie, follikulärem Lymphom, multiplem Myelom, Marginalzonenlymphom, lymphoproliferativer Erkrankung nach Transplantation (PTLD), HIV-assoziiertem Lymphom, Waldenstrom's Makroglobulinämie und primärem ZNS-Lymphom.

**Revendications**

1. Formulation, qui est sous une forme lyophilisée et qui comprend:

10 mg/ml de l'anticorps B-Ly 1 humanisé comprenant VH B-HH6 et VL B-KV du document de brevet WO2005/044859,
0,02 % de polysorbate 20 en m/v,
de la L-histidine 20 mM, et
du tréhalose 240 mM,
à pH 6,0.

2. Formulation, qui comprend:

25 mg/ml de l'anticorps B-Ly 1 humanisé comprenant VH B-HH6 et VL B-KV1 du document de brevet WO2005/044859,
0,02 % de Poloxamer 188™ en m/v,
de la L-histidine 20 mM, et
du tréhalose 240 mM,
à pH6,0;
ou
25 mg/ml de l'anticorps B-Ly 1 humanisé comprenant VH B-HH6 et VL B-KV1 du document de brevet WO2005/044859,
0,01 % de Poloxamer 188™ en m/v,
de la L-histidine 20 mM, et
du tréhalose 240 mM,
à pH 6,0 ;
ou
25 mg/ml de l'anticorps B-Ly 1 humanisé comprenant VH B-HH6 et VL B-KV du document de brevet WO2005/044859,
0,1 % de Poloxamer 188™ en m/v,
de la L-histidine 20 mM, et
du tréhalose 240 mM,
à pH 6,0 ;
ou
25 mg/ml de l'anticorps B-Ly 1 humanisé comprenant VH B-HH6 et VL B-KV1 du document de brevet WO2005/044859,
0,02 % de Polysorbate 80 en m/v,
de la L-histidine 20 mM, et
du tréhalose 240 mM,
à pH 6,0 ;
ou
30 mg/ml de l'anticorps B-Ly 1 humanisé comprenant VH B-HH6 et VL B-KV1 du document de brevet WO2005/044859,
0,01 % de Poloxamer 188™ en m/v,
de la L-histidine 20 mM, et
du tréhalose 200 mM,
à pH 6,5.

3.  Formulation selon la revendication 2, qui est sous forme liquide et qui comprend:

    25 mg/ml de l'anticorps B-Ly 1 humanisé comprenant VH B-HH6 et VL B-KV1 du document de brevet WO2005/044859,
    0,02 % de Poloxamer 188™ en m/v,
    de la L-histidine 20 mM, et
    du tréhalose 240 mM,
    à pH 6,0.

4.  Utilisation d'une formulation selon l'une quelconque des revendications 1 à 3 pour la préparation d'un médicament utile pour le traitement de maladies associées au CD20.

5.  Utilisation selon la revendication 4, où la maladie est choisie dans le groupe constitué par les lymphomes non hodgkiniens à lymphocytes B (LNH), le lymphome à cellules du manteau (LCM), la leucémie lymphoïde aiguë (LLA), la leucémie lymphoïde chronique (LLC), le lymphome diffus à grandes cellules à lymphocytes B (LDGC-B), le lymphome de Burkitt, la leucémie à tricholeucocytes, le lymphome folliculaire, le myélome multiple, le lymphome de la zone marginale, le syndrome lymphoprolifératif post-transplantation (SLPT), le lymphome associé au VIH, la macroglobulinémie de Waldenstrôm et le lymphome primaire du SNC.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5202238 A
- US 5204244 A
- WO 9411026 A
- WO 2005103081 A
- WO 2004035607 A
- WO 2004056312 A
- WO 2005044859 A
- US 6602684 B
- WO 2004065540 A

- WO 99154342 A
- US 3773919 A
- WO 9960023 A
- WO 2006099667 A
- US 2007027135 A
- US 20060241056 A
- WO 2004004749 A
- US 5780454 A

### Non-patent literature cited in the description

- **VALENTINE et al.** *J. Biol. Chem.,* 1989, vol. 264 (19), 11282-11287
- **EINFIELD et al.** *EMBO J.,* 1988, vol. 7 (3), 711-717
- **ANDERSON et al.** *Blood,* 1984, vol. 63 (6), 1424-1433
- **TEDDER et al.** *J, Immunol,* 1985, vol. 135 (2), 973-979
- **KOMAROMY et al.** *NAR,* 1983, vol. 11, 6775-6785
- **TEDDER et al.** *Eur. J. Immunol.,* 1986, vol. 25 (16), 881-887
- **TEDDER et al.** *J. Cell. Biochem.,* 1990, vol. 14D, 195
- **CRAGG, M.S. et al.** *Blood,* 2004, vol. 103, 2738-2743
- **CRAGG, M.S. et al.** *Blood,* 2003, vol. 101, 1045-1051
- **MORRISON, S.L. et al.** *Proc. Natl. Acad Sci. USA,* 1984, vol. 81, 6851-6855
- **RIECHMANN, L. et al.** *Nature,* 1988, vol. 332, 323-327
- **NEUBERGER, M.S. et al.** *Nature,* 1985, vol. 314, 268-270
- **VAN DIJK, M.A. ; VAN DE WINKEL, J.G.** *Curr. Opin. Pharmacol.,* 2001, vol. 5, 368-374
- **KELLERMANN, S. A. et al.** *Curr Opin Biotechnol,* 2002, vol. 13, 593-597
- **KABAT et al.** Sequences of Proteins of Immunological Interest. National Institutes of Health, 1991
- **PRESS et al.** *Blood,* 1987, vol. 69/2, 584-591
- **UMANA, P. et al.** *Nature Biotechnol.,* 1999, vol. 17, 176-180
- **REFF.** *Blood,* 1994, vol. 83 (2), 435-445

- **POPPEMA, S. ; VISSER, L.** *Biotest Bulletin,* 1987, vol. 3, 131-139
- **JENKINS et al.** *Nature Biotechnol.,* 1996, vol. 14, 975-81
- **CUMMING et al.** *Glycobiology,* 1991, vol. 1, 115-30
- **JENKINS et al.** *Nature Biotechnol.,* 1996, vol. 14, 975-981
- **WRIGHT, A. ; MONISON, S. L.** *Trends Biotech,* 1997, vol. 15, 26-32
- **WRIGHT, A. ; MORRISON, S. L.** *Trends Biotech,* 1997, vol. 15, 26-32
- **LIFELY, M. R. et al.** *Glycobiology,* 1995, vol. 5 (8), 813-22
- **LIFELY, M. R. et al.** *Glycobiology,* 1995, vol. 5, 813-822
- **JEFFERIS, R. et al.** *Immunol. Rev.,* 1998, vol. 163, 59-76
- **WRIGHT, A. ; MORRISON, S. L.** *Trends Biotechnol,* 1997, vol. 15, 26-32
- Remington's Pharmaceutical Sciences. 1980
- **COREY, S. et al.** *Cancer Cell,* 2005, 5-6
- **KISSELEV, A.L. et al.** *Chem Biol,* 2001, 739-758
- **JOAZEIRO, C. et al.** *Res,* 2006, vol. 66 (16), 7840-7842
- **KANAGASABAPHY et al.** *Curr Opin Investig Drugs,* 2007, vol. 8, 447-51
- **ADAMS, J.** *Nat Rev Cancer,* 2004, vol. 4, 349-360
- **ADAMS.** *Cur. Opin. Chem Biol.,* 2002, vol. 6, 493-500
- **MORAVEC et al.** *Cell Notes,* 2006, vol. 15, 4-7